# EUROPEAN PATENT SPECIFICATION

(11) **EP 1 718 774 B1**
(45) Date of publication and mention of the grant of the patent: **15.02.2012**
(21) Application number: 05717852.7
(22) Date of filing: 28.02.2005
(51) Int. Cl.: C12Q 1/70

(54) **DETECTION OF HUMAN PAPILLOMAVIRUS**
NACHWEIS VON MENSCHLICHEM PAPILLOMAVIRUS
DETECTION DU PAPILLOMAVIRUS HUMAIN

(30) Priority: 26.02.2004 GB 0404315
(43) Date of publication of application: 08.11.2006
(73) Proprietor: Norchip A/S, 3490 Klokkarstua (NO)
(72) Inventor: KARLSEN, Frank, Norchip A/S, N-3490 Klokkarstua (NO); WHITE, Nina Louise, Boult Wade Tennant, London WC1X 8BT (GB)
(74) Representative: White, Nina Louise
(86) International application number: PCT/GB2005/000774
(87) International publication number: WO 2005/083129

(56) References cited:
- WO-A-03/057914
- LANHAM S ET AL: "HPV detection and measurement of HPV 6, telomerase and survivin transcripts in colposcopy clinic patients" JOURNAL OF CLINICAL PATHOLOGY, LONDON, GB, vol. 54, no. 4, April 2001 (2001-04), pages 304-308, XP002961294 ISSN: 0021-9746
- SMITS H L ET AL: "APPLICATION OF THE NASBA NUCLEIC ACID AMPLIFICATION METHOD FOR THE DETECTION OF HUMAN PAPILLOMAVIRUS TYPE 16 E6-E7 TRANSCRIPTS" JOURNAL OF VIROLOGICAL METHODS, AMSTERDAM, NL, vol. 54, no. 1, 1995, pages 75-81, XP009015131 ISSN: 0166-0934
- JEON SAEWHA ET AL: "Integration of human papillomavirus type 16 DNA into the human genome leads to increased stability of E6 and E7 mRNAs: Implications for cervical carcinogenesis" PROCEEDINGS OF THE NATIONAL ACADEMY OF SCIENCES OF USA, NATIONAL ACADEMY OF SCIENCE. WASHINGTON, US, vol. 92, no. 5, 1995, pages 1654-1658, XP002260892 ISSN: 0027-8424
- O'LEARY: "Cervical Cancer Risk Evaluation Using the HPV-Detection Kit PreTect HPV-Proofer is a Strooke of Genius?"", , December 2001 (2001-12), Dublin
- "NorChip's PreTect® HPV-Proofer Demonstrates Ability to Detect HPV Oncogene Activity in Women at Risk for Developing Cervical Cancer", NORCHIP PRESS RELEASE, 3 July 2003 (2003-07-03), Klokkarstua, Norway
- CUSCHIERI ET AL.: "Human Papillomavirus Type Specific DNA and RNA Persistence - Implications for Cervical Disease Progression and Monitoring", JOURNAL OF MEDICAL VIROLOGY, vol. 73, 18 March 2004 (2004-03-18), page 65-70,

## Description

### Field of the invention

The present invention relates to *in vitro* methods of screening human subjects for the presence of human papillomavirus (HPV) which exhibits loss of regulation of E6/E7 mRNA expression and loss of replication and/or expression of a stabilized pre-mRNA encoding full length E6 protein. In particular, the invention provides *in vitro* methods of screening for persistent transforming HPV infection equivalent to persistent cell abnormalities or persistent CIN III lesions, cancer in situ or high-grade squamous intraepithelial lesions (HSIL). The methods are useful in the context of cervical cancer screening.

### Background to the invention

Cervical carcinoma is one of the most common malignant diseases world-wide and is one of the leading causes of morbidity and mortality among women (Parkin DM, Pisani P, Ferlay J (1993) Int J Cancer 54: 594-606;Pisani P, Parkin DM, Ferlay J (1993) Int J Cancer 55: 891-903). 15,700 new cases of invasive cervical cancer were predicted in the United States in 1996, and the annual world-wide incidence is estimated to be 450,000 by the World Health Organization (1990). The annual incidence rate differs in different parts of the world, ranging from 7.6 per 100,000 in western Asia to 46.8 per 100,000 in southern Africa (Parkin et al., 1993 *ibid*).

The current conception of cervical carcinoma is that it is a multistage disease, often developing over a period of 10-25 years. Invasive squamous-cell carcinoma of the cervix is represented by penetration through the basal lamina and invading the stroma or epithelial lamina propria. The clinical course of cervical carcinoma shows considerable variation. Prognosis has been related to clinical stage, lymph node involvement, primary tumour mass, histology type, depth of invasion and lymphatic permeation (Delgado G, et al., (1990) Gynecol Oncol 38: 352-357). Some patients with.less favourable tumour characteristics have a relatively good outcome, while others suffer a fatal outcome of an initially limited disease. This shows a clear need for additional markers to further characterise newly diagnosed cervical carcinomas, in order to administer risk-adapted therapy (Ikenberg H, et al., Int. J. Cancer 59:322-6. 1994).

The epidemiology of cervical cancer has shown strong association with religious, marital and sexual patterns. Almost 100 case-control studies have examined the relationship between HPV and cervical neoplasia and almost all have found positive associations (IARC monographs, 1995). The association is strong, consistent and specific to a limited number of viral types (Munoz N, Bosch FX (1992) HPV and cervical neoplasia: review of case-control and cohort studies. IARC Sci Publ 251-261). Among the most informative studies, strong associations with HPV 16 DNA have been observed with remarkable consistency for invasive cancer and high-grade CIN lesions, ruling out the possibility that this association can be explained by chance, bias or confounding (IARC monographs, 1995). Indirect evidence suggested that HPV DNA detected in cancer cells is a good marker for the role of HPV infection earlier in the carcinogenesis. Dose-response relationship has been reported between increasing viral load and risk of cervical carcinoma (Munoz and Bosch, 1992 *ibid*). In some larger series up to 100% of the tumours were positive for HPV but the existence of virus-negative cervical carcinomas is still debatable (Meijer CJ, et al., (1992) Detection of human papillomavirus in cervical scrapes by the polymerase chain reaction in relation to cytology: possible implications for cervical cancer screening. IARC Sci Publ 271-281; Das BC, et al., (1993) Cancer 72: 147-153).

The most frequent HPV types found in squamous-cell cervical carcinomas are HPV 16 (41%-86%) and 18 (2%-22%). In addition HPV 31, 33, 35, 39, 45, 51, 52, 54, 56, 58, 59, 61, 66 and 68 are also found (IARC, monographs, 1995). In the HPV2000 International conference in Barcelona HPV 16, 18, 31 and 45 were defined as high risk, while HPV 33, 35, 39, 51, 52, 56, 58, 59, 68 were defined as intermediate risk (Keerti V. Shah. P71). The 13 high risk plus intermediate risk HPVs are together often referred to as cancer-associated HPV types.

A number of studies have explored the potential role of HPV testing in cervical screening (see Cuzick et al. A systematic review of the role of human papillomavirus testing withing a cervical screening programme. Health Technol Assess 3:14. 1999).

Reid et al., (Reid R, et al., (1991) Am J Obstet Gynecol 164: 1461-1469) where the first to demonstrate a role for HPV testing in a screening context. This study was carried out on high-risk women from sexually transmitted disease clinics and specialist gynaecologists, and used a sensitive (low stringency) Southern blot hybridisation for HPV detection. A total of 1012 women were enrolled, and cervicography was also considered as a possible adjunct to cytology. Twenty-three CIN II/III lesions were found altogether, but only 12 were detected by cytology (sensitivity 52%, specificity 92%). HPV testing found 16 high-grade lesions.

Bauer et al. (Bauer HM, et al., (1991) JAMA 265: 472-477) report an early PCR-based study using MY09/11 primers (Manos M, et al.,(1990) Lancet 335: 734) in young women attending for routine smears (college students). They found a positive rate of 46% in 467 women, which was much higher than for dot blot assay (11%).

In a study using PCR with GP5/6 primers (Van Den Brule AJ, et al., (1990) J Clin Microbiol 28: 2739-2743) van der Brule et al. (Van Den Brule AJ, et al., (1991) Int J Cancer 48: 404-408) showed a very strong correlation of HPV positivity with cervical neoplasia as assessed by cytology. In older women (aged 35-55 years) with negative cytology the HPV positivity rate was only 3.5%, and this was reduced to 1.5% if only types 16, 18, 31 and 33 were considered, while women with histological carcinoma in situ were all HPV-positive, and 90% had one of the four above types. Women with less severe cytological abnormalities had lower HPV positivity rates in a graded way, showing a clear trend.

Roda Housman et al. (Roda Housman AM, et al., (1994) Int J Cancer 56: 802-806) expanded these observations by looking at a further 1373 women with abnormal smears. This study also confirmed increasing positivity rate with increasing severity of smear results. They also noted that the level of HPV heterogeneity decreased from 22 types for low-grade smears to ten "high-risk" types for high grade smears. This paper did not include any cytologically negative women, nor was cytological disease confirmed histologically.

Cuzick et al. (Cuzick J, et al., (1992) Lancet 340: 112-113; Cuzick J, et al., (1994) Br J Cancer 69: 167-171) were the first to report that HPV testing provided useful information for the triage of cytological abnormalities detected during random screening. In a study of 133 women, referral for coloposcopy they found a positive predictive value of 42%, which was similar to that for moderate dyskaryosis. The results were most striking for HPV 16, where 39 of 42 HPV 16 positive women were found to have high-grade CIN on biopsy. This study pointed out the importance of assessing viral load and only considered high levels of high-risk types as positive.

Cox et al. (Cox JT, et al., (1995) Am J Obstet Gynecol 172: 946-954) demonstrated a role for HPV testing using the Hybrid Capture^{™} system (DIGENE Corporation, Gaithersburg, MD, USA) for triaging women with borderline smears. This test was performed on 217 such women from a college referral service, and a sensitivity of 93% was found for CINII/III compared with 73% for repeat cytology. High viral load was found to further improve performance by reducing false positives. When 5 RLU was taken as a cut-off, a PPV of approximately 24% was found with no loss of sensitivity.

WO 91/08312 describes methods for determining the prognosis of individuals infected with HPV which comprise measuring the level of HPV activity by detecting transcripts of all or a portion of the E6 and/or E7 HPV genes in a sample and comparing the measurements of HPV activity with a previously established relationship between activity and risk of progression to serious cervical dysplasia or carcinoma.

WO 99/29890 describes methods for the assessment of HPV infection based on the measurement and analysis of gene expression levels. In particular, WO 99/29890 describes methods which are based on measuring the levels of expression of two or more HPV genes (e.g. HPV E6, E7, L1 and E2) and then comparing the ratio of expression of combinations of these genes to provide an indication of the stage of HPV-based disease in a patient.

The present inventors have previously determined that it is possible to make a clinically useful assessment of HPV-associated disease based only on a simple positive/negative determination of expression of E6/E7 mRNA transcripts, with no requirement for accurate quantitative measurements of expression levels. This method is technically simple and, in a preferred embodiment, is amenable to automation in a mid-to-high throughput format. This method is described in detail in the applicant's published International application WO 03/57914.

The method described in WO 03/57914 is preferably carried out using the Pre-Tect HPV-Proofer^{™} kit, which is commercially available from Norchip AS. The HPV-Proofer assay provides three levels of information:
(1) Identification of mRNA from five specific different HPV-types (16, 18, 31, 33 and 45);
(2) Determination of the presence of oncogene HPV E6/E7 mRNA; and
(3) Determination of the presence of full length E6/E7 mRNA indicating dysregulation.

Each sample undergoes three duplex NASBA reactions, therefore six results are reported for each sample. Negative controls are included each time to monitor contamination. Positive controls are included for all HPV types to monitor reagent performance. Intrinsic cellular control U1A mRNA (cellular housekeeping gene) monitors entire test procedure to eliminate possible false negatives. It is not possible for the HPV-Proofer assay to detect HPV DNA. PreTect Analysis Software (PAS) is used for automated routine data analysis, interpretation and reporting.

The utility of the HPV-Proofer assay has been evaluated in at least 12 clinical studies.

The present inventors have now determined that the Pre-Tect HPV-Proofer^{™} assay does not detect HPV virions, even though it detects HPV-mRNA from HPV 16, 18, 31, 33 and 45. Rather, presence of E6/E7 transcript, as may be determined with the HPV-Proofer assay, is indicative of a loss of transcriptional regulation and loss of ability to replicate, and/or expression of a stabilised E6/E7 pre-mRNA which encodes full length E6 protein. It is impossible for the Pre-Tect HPV-Proofer to detect infectious virus HPV particles, since the virus cannot produce virions when transcriptional regulation is lost and the virus is integrated. The viruses inside the cells detected as positive for E6/E7 expression using HPV-Proofer have left their normal life-cycles and lost their regulation of either transcription control of the promoters from all the E6/E7 transcripts, or lost the splicing capability, cleaving the E6/E7 transcript behind or else express a stabilised form of the full length pre-mRNA which encodes the full length E6 protein.

The inventors have also observed that the expression of E6/E7 mRNA transcripts in HPV-infected cells correlates with cellular changes characterised as the presence of enlarged cell nuclei, aneuploidy (typically more than 5 or 9 centromeres per cell) and also mitosis. Cells that are positive for E6/E7 expression (e.g. using the PreTect HPV-Proofer test) have something wrong, they exhibit cell abnormalities or have large maturated cell nuclei. These results also correlate with cytological and histological characterisation of cervical lesions. Cytologically or histologically defined low-grade lesions lacking cells with enlarged cell nuclei and with less than 9 centromeres do not give positive results for expression of E6/E7 mRNA expression with HPV-Proofer.

Therefore, the inventors have determined that expression of E6/E7 transcripts of human papillomavirus can be used as a molecular indicator of the presence of cellular abnormalities associated with the presence of a persistent infection with human papillomavirus. Detection of E6/E7 expression may therefore be used to distinguish between high and low grade cervical lesions. In particular, detection of E6/E7 expression can discriminate between histologically-defined CIN III samples without aneuploidic cells and those having aneuploidic cells; samples positive for E6/E7 mRNA encoding the full length E6 protein are scored as having aneuploidic cells. Detection of E6/E7 expression can also distinguish between histologically defined CIN III or CIN II (HSIL cases) cases that go into regress and those in which infection persists or progresses; samples positive for E6/E7 mRNA encoding the full length E6 protein are scored as having an infection likely to progress if left untreated.

The present inventors have still further concluded that:
(1) Incidence of expression from E6/E7 oncogenes increases with the severity of the lesion.
(2) Detecting HPV oncogenic activity by assessment of E6/E7 expression, optionally in combination with HPV typing, is a powerful predictor of high-grade lesions.
(3) The significant majority of cervical cancers (96%) contain at least one of the five main carcinogenic HPV-types (HPV 16, 18, 31, 33 and 45).
(4) Those who tested positive for E6/E7 expression with Pre-Tect HPV-Proofer^{™} were significantly more likely to maintain a persistent infection than those without positive HPV-Proofer results.

Accordingly, a first aspect discloses an *in vitro* method of screening human subjects for the presence of human papillomavirus in at least one cell or tissue, wherein the human papillomavirus exhibits loss of regulation of E6/E7 mRNA expression and loss of replication and/or expression of a stabilized pre-mRNA which encodes a full length E6 protein, the method comprising detecting the presence of mRNA transcripts of the E6/E7 gene of a human papillomavirus which encode full length E6 protein in a test sample comprising mRNA derived from the cell or tissue, wherein the presence of such E6/E7 mRNA transcripts in the sample is taken as an indication of the presence of human papilloma virus exhibiting loss of regulation of E6/E7 mRNA expression and loss of replication and/or expression of a stabilized pre-mRNA encoding full length E6 protein in the cell or tissue.

In a second aspect the application discloses an *in vitro* method of screening human subjects for the presence of cellular changes characterized by enlarged cell nuclei and cellular aneuploidy in at least one cell or tissue, which method comprises detecting the presence of mRNA transcripts of the E6/E7 gene of human papillomavirus which encode a full length E6 protein in a test sample comprising mRNA derived from the cell or tissue, wherein the presence of such E6/E7 mRNA transcripts in the sample is taken as an indication that the cell or tissue under test exhibits the cellular changes.

In a third aspect the invention provides an in vitro method of screening human subjects for the presence of persistent transforming infection with human papillomavirus in at least one cell or tissue, which method comprises screening the subject for expression of mRNA transcripts of the E6/E7 gene of human papillomavirus which encode a full length E6 protein in a test sample comprising mRNA derived from the cell or tissue, wherein subjects positive for expression of such mRNA transcripts of the E6/E7 gene of human papillomavirus are scored as having a persistent transforming infection with human papillomavirus in the cell or tissue.

A "persistent transforming infection" with one of HPV types 16, 18, 31, 33 or 45, identified by the presence of E6/E7 mRNA transcripts encoding full length E6 protein from one of these HPV subtypes, is considered to be equivalent to persistent cell abnormalities or persistent CIN III lesions, cancer in situ or high-grade squamous intraepithelial lesions (HSIL) as assessed by cytology or histology. Therefore, the method of the third aspect of the invention provides a method of screening for persistent transforming infection with human papillomavirus equivalent to persistent cell abnormalities or persistent CIN III lesions; cancer in situ or high-grade squamous intraepithelial lesions (HSIL).

Persistent transforming HPV infection as defined by the persistent presence of E6/E7 HPV mRNA encoding full length E6 protein may directly correlate with persistent CIN II+ and therefore serve as a prognostic marker in the cervical screening program. In particular, the method of the invention may be used in the triage of women identified as having atypical squamous cells of undetermined significance (ASCUS) or low-grade squamous intraepithelial lesion (LSIL) on the basis of cytology/histology. Management of such patients is problematic because only a small proportion will progress to cervical intraepithelial neoplasia (CIN) III and invasive cervical carcinoma (ICC). Follow-up testing by cytology/histology fails to identify all those women at higher risk of CIN II+. In the clinical studies reported herein detection of expression of full length E6/E7 mRNA encoding full length E6 protein exhibited marked specificity for high grade cervical lesions, indicating that the presence of such transcripts provides a useful prognostic marker.

A positive screening result in the methods of the invention is indicated by detection of expression of E6/E7 mRNA transcripts which encode a full length E6 protein. A positive result for E6/E7 mRNA expression indicates that the subject carries virus which exhibits loss of regulation of E6/E7 expression and/or which expresses a stabilised pre-mRNA encoding a full length E6 protein and is further indicative that the subject has abnormal cell changes.

The term "loss of E6/E7 regulation" as used herein means a loss of regulation of either transcription control of the promoters from all the E6/E7 transcripts, or a loss of the normal splicing capability in the E6/E7 open reading frames.

The term "stabilised pre-mRNA" refers to a primary E6/E7 transcript which has not undergone any splicing event within the E6 open reading frame and hence encodes a full length protein and is more stable (i.e exhibits a longer half-life) than any equivalent primary (unspliced) E6/E7 transcript encoding full length E6 protein expressed by a native or wild-type human papillomavirus of the same HPV sub-type. Native or wild type human papillomavirus refers to virus which has no genomic modification associated with persistent infection of a human host and is not integrated into the human genome. Such native and wild type viruses are also characterised in that the splicing of E6/E7 pre-mRNA or full-length mRNA transcripts occurs very soon after transcription, such that the full length pre-mRNA does not accumulate, within the cell to any significant extent. Thus, the pre-mRNA is generally not translated to give full length E6 protein in cells infected with native or wild type virus because the pre-mRNA is processed by the splicing apparatus before translation can take place. However, in a cell with abnormalities associated with persistent transforming HPV infection the pre-mRNA is stabilised and/or not spliced and can therefore accumulate within the cell to a level that is not observed in normal cells or normal proliferating cells, e.g. cells infected with replicating HPV.

Relative to primary E6/E7 primary (unspliced) transcripts expressed in native or wild type virus, the stabilised pre-mRNA may be modified, for example by addition of mRNA sequences transcribed from the human genome as a result of viral integration or by deletion of HPV sequences, in a region of the transcript outside of the open reading frame encoding the E6 protein. Hence the stabilised pre-mRNA may be of different sequence and structure to the E6/E7 primary (unspliced) transcripts expressed in native or wild type virus but will still encode a full length E6 protein.

The term "abnormal cell changes" encompasses cell changes which are characteristic of more severe disease than low-grade cervical lesions or low squamous intraepithelial lesions, including cell changes which are characteristic of disease of equal or greater severity than high-grade CIN (defined as a neoplastic expansion of transformed cells), CIN (cervical intraepithelial neoplasia) III, or high squamous intraepithelial neoplasia (HSIL), including lesions with multiploid DNA profile and "malignant" CIN lesions with increased mean DNA-index values, high percentage of DNA-aneuploidy and 2.5c Exceeding Rates (Hanselaar et al., 1992, Anal Cell Pathol., 4:315-324; Rihet et al., 1996, J. Clin Pathol 49:892-896; and McDermott et al., 1997, Br. J. Obstet Gynaecol. 104:623-625).

Cervical Intraepithelial Neoplasia (abbreviated "CIN"), also called Cervical Dysplasia, is a cervical condition caused Human Papilloma Virus. CIN is classified as I, II or III depending on its severity. It is considered a pre-cancerous abnormality, but not an actual cancer. The mildest form, CIN I, usually goes away on its own, although rarely it can progress to cancer. The more severe forms, CIN II and CIN III, most often stay the same or get worse with time. They can become a cancer, but almost never do if treated adequately.

HPV has been identified as a causative agent in development of cellular changes in the cervix, which may lead to the development of cervical carcinoma. These cellular changes are associated with constitutive or persistent expression of E6/E7 proteins from the HPV viral genome. Thus, it is possible to conclude that subjects in which expression of E6/E7 mRNA can be detected, particularly those subjects who exhibit persistent E6/E7 expression when assessed over a period of time, already manifest cellular changes in the cervix. These changes may have taken place in only a very few cells of the cervix, and may not be detectable by conventional cytology. Nevertheless, with the use of sensitive, specific and accurate methods for detection of E6/E7 mRNA it is possible to identify those subjects who already exhibit cellular changes in the cervix at a much earlier stage than would be possible using conventional cytological screening. This will allow earlier intervention with treatments aimed at preventing the development of cervical carcinoma.

As a result of HPV integration into the human genome or as a result of the "modification" in a modified episomal HPV genome, normal control of the viral E6/E7 oncogene transcription is lost (Durst et al., 1985, J Gen Virol, 66(Pt 7): 1515-1522; Pater and Pater, 1985 Virology 145:313-318; Schwarz et al., 1985, Nature 314: 111-114; Park et al., 1997, ibid). In contrast, in premalignant lesions and HPV-infected normal epithelium papillomaviruses predominate in "unmodified" episomal forms, hence oncogene (E6/E7) transcription may be absent or efficiently down-regulated (Johnson et al., 1990, J Gen Virol, 71(Pt 7): 1473-1479; Falcinelli et al., 1993, J Med Virol, 40: 261-265). Integration of human papillomavirus type 16 DNA into the human genome is observed to lead to a more unstable cell activity/genome, and increased stability of E6 and E7 mRNAs (Jeon and Lambert, 1995, Proc Natl Acad Sci USA 92: 1654-1658). Thus HPV integration, typically found in cervical cancers but only infrequently found in CIN lesions (Carmody et al., 1996, Mol Cell Probes, 10: 107-116), appears to be an important event in cervical carcinogenesis.

In a clinical context the performance of methods which rely on screening for expression of E6/E7 mRNA alone is critically dependent on the ability to score a negative result for E6/E7 mRNA expression with confidence. This again requires a detection technique which has maximal sensitivity, yet produces minimal false-negative results. In a preferred embodiment this is achieved by using a sensitive amplification and real-time detection technique to screen for the presence or absence of E6/E7 mRNA. The most preferred technique is real-time NASBA amplification using molecular beacons probes, as described by Leone et al., Nucleic Acids Research., 1998, Vol 26, 2150-2155. Due to the sensitivity of this technique the occurrence of false-negative results is minimised and a result of "negative E6/E7 expression" can be scored with greater confidence. This is extremely important if the assays are to be used in the context of a clinical screening program.

It is preferred to assay for expression of E6/E7 mRNA transcripts from any one or more of (and more preferably all of) HPV types 16, 18, 31, 33 and 45. In one embodiment the assay may detect only these HPV types. DNA from HPV types 16, 18, 31 and 33, has been detected in more than 96% of cervical carcinoma samples in a Norwegian study population. Other studies have shown that E6 and E7 are almost invariably retained in cervical cancers, as their expression is likely to be necessary for conversion to and maintenance of the malignant state (Choo et al., 1987, J Med Virol 21:101-107; Durst et al., 1995, Cancer Genet Cytogenet, 85: 105-112). In contrast to HPV detection systems which are based on detection of the undamaged genome or the L1 gene sequence, detection of HPV mRNA expressed from the E6/E7 area may detect more than 90% of the patients directly related to a risk of developing cervical carcinoma.

In the clinic, methods based on detection of E6/E7 mRNA may be used in post-screening or triage, i.e. further analysis of individuals having a previous diagnosis of ASCUS, CIN 1 or Condyloma. The method may be used to select those with a high risk of developing cervical carcinoma from amongst the group of individuals having a previous diagnosis of ASCUS, CIN 1 or Condyloma. ASCUS, Condyloma and CIN I may be defined as more or less the same diagnosis due to very low reproducibility between different cytologists and different cytological departments. Östör (Int J. Gyn Path. 12:186-192. 1993) found that only around 1% of -the CIN 1 cases may progress to cervical carcinoma. Thus, there is a genuine need for an efficient method of identifying the subset of individuals with ASCUS, Condyloma or CIN I who are at substantial risk of developing cervical carcinoma. One of HPV types 16, 18, 31 or 33 was detected in 87% of the cervical carcinoma cases study by Karlsen *et al*., 1996. By inclusion of HPV 45, nearly 90% of the cervical carcinoma samples are found to be related to these five HPV types. Therefore, calculated from the data provided by Östör (Int J. Gyn Path. 12:186-192. 1993) more than 99.9% are detected cases with ASCUS, CIN I or condyloma are missed by our HPV-Proofer kit.

The high sensitivity and specificity of the present method means that it may find utility in primary screening, reflex-testing kit or routine diagnostics for detection of women with a high or very high risk of developing cervical carcinoma.

In the method of the invention "positive expression" of an mRNA is taken to mean expression above background. There is no absolute requirement for accurate quantitative determination of the level of E6/E7 mRNA expression.

In certain embodiments, the methods of the invention may comprise a quantitative determination of levels of mRNA expression. In a preferred embodiment in order to provide a clear distinction between "positive expression" and "negative expression" a determination of "positive expression" may require the presence of more than 50 copies of the relevant mRNA (per ml of sample or per total volume of sample), whereas a determination of "negative expression" may require the presence of less than 1 copy of the relevant mRNA (per ml of sample or per total volume of sample).

The methods of the invention will preferably involve screening for E6/E7 mRNA using a technique which is able to detect specifically E6/E7 mRNA from cancer-associated HPV types, more preferably "high risk" cancer-associated HPV types. In the most preferred embodiment the methods involve screening for E6/E7 mRNA using a technique which is able to detect E6 mRNA from HPV types 16, 18, 31 and 33, and preferably also 45. Most preferably, the method will specifically detect expression of E6/E7 mRNA from at least one of HPV types 16, 18, 31, 33, and preferably also 45, and most preferably all five types. However, women positive for positive for expression of E6/E7 from other types than 16, 18, 31, 33 and 45, e.g. 35, 39, 45, 52, 56, 58, 59, 66 and 68 may still manifest cellular abnormalities. Thus, the method may encompass screening for expression of E6/E7 mRNA from one or more of these HPV types, most preferably in addition to screening for E6/E7 mRNA from HPV types 16, 18, 31, 33 and 45. Certain HPV types exhibit a marked geographical/population distribution. Therefore, it may be appropriate to include primers specific for an HPV type known to be prevalent in the population/geographical area under test, for example in addition to screening for HPV types 16, 18, 31, 33 and 45.

The methods of the invention are based on detection of full length E6/E7 mRNA transcripts of some or all of the HPV types which encode a full length E6 protein. In these embodiments presence of the full length E6/E7 mRNA is taken as a positive screening result.

The term "full length E6/E7 mRNA transcripts" excludes any of the naturally occurring splice variants, but encompasses bicistronic transcripts that encode functional full length E6 and E7 proteins. Four E6/E7 mRNA species have so far been described in cells infected with HPV 16, namely an unspliced E6 transcript and three spliced transcripts denoted E6*I, E6*II and E6*III (Smotkin D, et al., J Virol. 1989 Mar 63(3):1441-7; Smotkin D, Wettstein FO. Proc Natl Acad Sci USA. 1986 Jul 83(13):4680-4; Doorbar J. et al., Virology. 1990 Sep 178(1) :254-62; Cornelissen MT, et al. J Gen Virol. 1990 May 71(Pt 5):1243-6; Johnson MA, et al. J Gen Virol. 1990 Jul 71(Pt 7):1473-9; Schneider-Maunoury S, et al. J Virol. 1987 Oct 61(10):3295-8; Sherman L, et al. Int J Cancer. 1992 Feb 50(3):356-64). All four transcripts are transcribed from a single promoter (p97) located just upstream of the second ATG of the E6 ORF. In the case of HPV 16, the term "full length E6/E7 transcripts" refers to transcripts which contain all or substantially all of the region from nucleotide (nt) 97 to nt 880 in the E6 ORF, inclusive of nt 97 and 880. Nucleotide positions are numbered according to standard HPV nomenclature (see Human Papillomavirus Compendium OnLine, available via the internet or in paper form from HV Database, Mail Stop K710, Los Alamos National Laboratory, Los Alamos, NM 87545, USA).

In relation to HPV types other than HPV 16, "full length" E6/E7 transcripts may be taken to include transcripts which contain sequences homologous to the above-stated region of the HPV 16 E6/E7 transcript and to exclude E6 splice variants. Various sequence alignments of HPV types are publicly available via the Human Papillomavirus Compendium OnLine.

Specific detection of full length E6/E7 mRNA transcripts may be accomplished, for example, using primers or probes which are specific for the region which is present only in full length E6/E7 transcripts, not in splice variants.

The E6*I transcript exhibits loss of a coding sequence between nucleotides 226 and 409 (in HPV type 16) and the E*6II transcript exhibits loss of the coding sequence between nucleotides 226 and 526 (in HPV type 16). It is therefore preferred to use at least one primer or probe from the region located between nucleotides 226 and 409 of HPV type 16 or the homologous region from one of HPV types 18, 31, 33 or 45. Specificity for full length transcripts can be achieved by the use of a primer-pair in which one primer is specific for a sequence located within this region and the other primer is specific for a sequence located outside of this region or wherein both primers are specific for sequences within this region, preferably in conjunction with a probe specific for a sequence located within this region. In other embodiments it may be possible to use a primer-pair in which both primers are specific for sequences outside this region in combination with a probe specific for a sequence within the region in order to confer specificity for mRNA encoding full length E6.

Different HPV types exhibit different patterns of E6/E7 mRNA expression. Transcript maps for various HPV types, including HPV types 16 and 31, which may be used to assist in the design of probes or primers for detection of full length E6/E7 transcripts are publicly available via the Human Papillomavirus

Compendium (as above).

### Assay methodology

The methods of the invention involve screening for the presence of E6/E7 transcripts in at least one cell or tissue, and more particularly in a sample comprising RNA from at least one cell or tissue. The at least one cell or tissue must comprise at least one cervical cell of a type which is susceptible to infection with human papillomavirus, i.e. cervical epithelial cells.

The disclosed screening methods may be carried out on a preparation of nucleic acid isolated from a clinical sample or biopsy containing cervical cells taken from the subject under test. Suitable samples which may be used as a source of nucleic acid include (but not exclusively) cervical swabs, cervical biopsies, cervical scrapings, samples removed with the use of brushes and tampons etc., skin biopsies/warts, also paraffin embedded tissues, and formalin or methanol fixed cells.

The preparation of nucleic acid to be screened using the disclosed methods must include mRNA, however it need not be a preparation of purified poly A+ mRNA and preparations of total RNA or crude preparations of total nucleic acid containing both RNA and genomic DNA, or even crude cell lysates are also suitable as starting material for a NASBA reaction. Essentially any technique known in the art for the isolation of a preparation of nucleic acid including mRNA may be used to isolate nucleic acid from a test sample. A preferred technique is the "Boom" isolation method described in US-A-5,234,809 and EP-B-0389,063. This method, which can be used to isolate a nucleic acid preparation containing both RNA and DNA, is based on the nucleic acid binding properties of silicon dioxide particles in the presence of the chaotropic agent guanidine thiocyanate (GuSCN).

The methods of the invention are based on assessment of active transcription of the HPV genome. The methods are not limited with respect to the precise technique used to detect mRNA expression. Many techniques for detection of specific mRNA sequences are known in the art and may be used in accordance with the invention. For example, specific mRNAs may be detected by hybridisation, amplification or sequencing techniques.

It is most preferred to detect mRNA expression by means of an amplification technique, most preferably an isothermal amplification such as NASBA, transcription-mediated amplification, signal-mediated amplification of RNA technology, isothermal solution phase amplification, etc. All of these methods are well known in the art More preferably mRNA expression is detected by an isothermal amplification in combination with real-time detection of the amplification product. The most preferred combination is amplification by NASBA, coupled with real-time detection of the amplification product using molecular beacons technology, as described by Leone et al., Nucleic Acids Research, 1998, Vol 26, 2150-2155.

Methods for the detection of HPV in a test sample using the NASBA technique will generally comprise the following steps:
(a) assembling a reaction medium comprising suitable primer-pairs, an RNA directed DNA polymerase, a ribonuclease that hydrolyses the RNA strand of an RNA-DNA hybrid without hydrolysing single or double stranded RNA or DNA, an RNA polymerase that recognises said promoter, and ribonucleoside and deoxyribonucleoside triphosphates;
(b) incubating the reaction medium with a preparation of nucleic acid isolated from a test sample suspected of containing HPV under reaction condition which permit a NASBA amplification reaction; and
(c) detecting and/or quantitatively measuring any HPV-specific product of the NASBA amplification reaction.

Detection of the specific product(s) of the NASBA reaction (i.e. sense and/or antisense copies of the target RNA) may be carried out in a number of different ways. In one approach the NASBA product(s) may be detected with the use of an HPV-specific hybridisation probe capable of specifically annealing to the NASBA product. The hybridisation probe may be attached to a revealing label, for example a fluorescent, luminescent, radioactive or chemiluminescent compound or an enzyme label or any other type of label known to those of ordinary skill in the art. The precise nature of the label is not critical, but it should be capable of producing a signal detectable by external means, either by itself or in conjunction with one or more additional substances (e.g. the substrate for an enzyme).

A preferred detection method is so-called "real-time NASBA" which allows continuous monitoring of the formation of the product of the NASBA reaction over the course of the reaction. In a preferred embodiment this may be achieved using a "molecular beacons" probe comprising an HPV-specific sequence capable of annealing to the NASBA product, a stem-duplex forming oligonucleotide sequence and a pair of fluorescer/quencher moieties, as known in the art and described herein. If the molecular beacons probe is added to the reaction mixture prior to amplification it may be possible to monitor the formation of the NASBA product in real-time (Leone et al., Nucleic Acids Research, 1998, Vol 26, 2150-2155). Reagent kits and instrumentation for performing real-time NASBA detection are available commercially (e.g. NucliSens^{™} EasyQ system, from Organon Teknika).

In a further approach, the molecular beacons technology may be incorporated into the primer 2 oligonucleotide allowing real-time monitoring of the NASBA reaction without the need for a separate hybridisation probe.

In a still further approach the products of the NASBA reaction may be monitored using a generic labelled detection probe which hybridises to a nucleotide sequence in the 5' terminus of the primer 2 oligonucleotide. This is equivalent to the "NucliSens^{™}" detection system supplied by Organon Teknika. In this system specificity for NASBA products derived from the target HPV mRNA may be conferred by using HPV-specific capture probes comprising probe oligonucleotides as described herein attached to a solid support such as a magnetic microbead. Most preferably the generic labelled detection probe is the ECL^{™} detection probe supplied by Organon Teknika. NASBA amplicons are hybridized to the HPV-specific capture probes and the generic ECL probe (via a complementary sequence on primer 2). Following hybridization the bead/amplicon/ECL probe complexes may be captured at the magnet electrode of an automatic ECL reader (e.g. the NucliSens^{™} reader supplied by Organon Teknika). Subsequently, a voltage pulse triggers the ECL^{™} reaction.

Preferred embodiments of the method rely on amplification of E6/E7 mRNA from at least the major cancer-associated HPV types 16, 18, 31 and 33, and preferably also HPV 45. There are several different ways in which this can be achieved.

In one embodiment, separate primer-pairs specific for each of HPV types 16, 18, 31 and 33, and preferably also HPV 45 may be used to amplify transcripts from each HPV type individually. Alternatively, mixtures of two or more primer-pairs in a single container may be used to enable multiplexing of the amplification reactions.

In a further embodiment, a single primer-pair capable of amplifying a region of the E6/E7 gene from HPV types 16, 18, 31 and 33, and preferably also HPV 45 may be used, which thus enables amplification of all four (preferably five) types in a single amplification reaction. This could, for example, be achieved with the use of a pair of degenerate primers or by selection of a region of the E6/E7 mRNA which is highly conserved across HPV types.

The E6/E7 primer-pair may correspond to any region of the E6/E7 mRNA, and may enable amplification of all or part of the E6 open reading frame and/or the E7 open reading frame. Preferably it will enable amplification of full length transcripts which encode a full length E6 protein.

In a further approach, specificity for multiple HPV types may be achieved with the use of degenerate oligonucleotide primers or complex mixtures of polynucleotides which exhibit minor sequence variations, preferably corresponding to sites of sequence variation between HPV genotypes. The rationale behind the use of such degenerate primers or mixtures is that the mixture may contain at least one primer-pair capable of detecting each HPV type.

In a still further approach specificity for multiple HPV types may be achieved by incorporating into the primers one or more inosine nucleotides, preferably at sites of sequence variation between HPV genotypes.

Lists of suitable primers and probes which may be used for the detection of E6/E7 mRNA from various HPV types may be found in WO 03/057914 and in WO 03/057927.

The method of the invention is preferably carried out using the Pre-Tect HPV-Proofer^{™} assay and kit. However, it is to be understood that the invention is not limited to the use of this specific assay.

The method of the present invention will score negative for real histological negative and representative samples from the whole or parts of cervix, corpus or/and the cervical canal. This provides the outstanding specificity that makes the PreTect HPV-Proofer^{™} one of the most promising primary screening methods ever developed.

The specificity of using PreTect HPV-Proofer^{™} alone for diagnostics of women at risk of developing cervical carcinoma has been proved to be independent of age and works with more than three times higher specificity than a commercial DNA-based assay alone.

Detection of E6/E7 transcripts has the potential to identify which high-risk infections may persist without having to perform repeat testing. Incidence of expression from E6/E7 oncogenes increases with the severity of the lesion.

The methods of the invention may be performed in combination with HPV genotyping by any suitable method. The term "HPV genotyping" refers to any technique which enables identification of the HPV subtype(s) present in a given individual. Assessment of HPV oncogenic activity by detection of E6/E7 expression in combination with HPV typing promises to be a powerful predictor of high-grade lesions.

The invention will be further understood with reference to the following experimental examples.

### Pre-Tect HPV Proofer^{™} Assay

For all experimental examples which refer to the use of the Pre-Tect HPV Proofer^{™} kit and assay, the assay was performed using the commercially available kit according to the supplied instructions. Further information concerning the operation of the assay for real-time detection of HPV E6/E7 mRNA may be found in WO 03/057914.

The following experimental section summarises clinical data obtained using the Pre-Tect HPV-Proofer^{™} assay and kit.

### Example 1 - E6 and E7 mRNA expression from carcinogenic human papillomavirus (HPV) in 4136 cervical samples collected from an outpatient population

The aim of this study was to identify the presence of E6/E7 mRNA and DNA in cytological HGSIL/CIN3 samples confirmed by histology.

### Material and Methods:

The samples were collected from a well-screened outpatient population, including women older than 30 years of age (n=4136). E6/E7 transcripts from each of the high-risk HPV types 16, 18, 31, 33, and 45 were detected by the PreTect HPV-Proofer assay (NorChip AS, Klokkarstua, Norway), based on real-time multiplex NASBA. The presence of HPV DNA was investigated by Gp5+/6+ consensus PCR, and HPV DNA positive samples were then subjected to type specifc PCR for HPV types 16, 18, 31, 33 and 45. Women with a cytological HGSIL diagnosis were referred to biopsy and histology.

Histologically confirmed cases were registered at the Norwegian Cancer Registry. In Norway, cytological HGSIL can be divided into HGSIL/AGUS, HGSIL/ASC-H, HGSIL/CIN2, and HGSIL/CIN3.

### Results:

Of 25 cytological HGSIL cases, 14 were by histology confirmed as CIN2+. Two histological CIN2+ cases were by cytology diagnosed as HGSIL/ASC-H and HGSIL/CIN2. PreTect HPV Proofer detected 52% (13/25) of the cytological HGSIL cases, 86% (12/14) of the histological CIN2+ cases, and 9% (1/11) of the cytological HGSIL cases not verified by histology. The numbers for Gp5+/6+ PCR are 64% (16/25), 93% (13/14), and 27% (3/11), respectively. The one histological CIN2+ sample positive by consensus PCR, yet negative by PreTect HPV-Proofer, was identified as HPV 35. The prevalence of HGSIL/CIN3 was 0.29% (12/4136) and the prevalence of histological CIN2+ was 56% (14/25). Sensitivity, specificity, and positive and negative predictive values for PreTect HPV-Proofer and consensus PCR, are given in Table 1.

**Table 1:**

| | Total (n=4136) endpoint cytological HGSIL/CIN3 | | Cytological HGSIL (n=25) endpoint cytological CIN2+ | |
|---|---|---|---|---|
| | HPV Proofer | consensus PCR | HPV Proofer | consensus PCR |
| sensitivity | 75.0% | 83.3% | 85.7% | 92.9% |
| specificity | 97.2% | 89.9% | 90.9% | 72.7% |
| PPV | 7.3% | 2.3% | 92.3% | 81.3% |
| NPV | 97.2% | 99.9% | 83.3% | 88.9% |

| | | | | |
|---|---|---|---|---|
| PPV=Positive Predictive Value NPV=Negative Predictive Value | | | | |

### Discussion and Conclusion:

There was a good agreement between cytological HGSIL/CIN3 and histological CIN2+ cases, with only one cytological HGSIL/CIN3 not confirmed as CIN2+ by histology. In histologically verified CIN2+ cases, the detection grade for both HPV DNA and mRNA were high, and nearly identical. In cytological HGSIL cases not verified by histology, the detection grade for-PreTect HPV-Proofer was lower than for consensus PCR. Together, cytological HGSIL/CIN3 and PreTect HPV-Proofer detected all histological CIN2+.

In conclusion, HPV E6/E7 transcripts from the five most frequently found carcinogenic HPV types, HPV 16, 18, 31, 33, and 45, seem to be present in nearly all histological CIN2+ cases. The high specificity and positive predictive value for PreTect HPV-Proofer is an advantage in HPV diagnostics and hence mRNA detection is a suitable supplement to cytology and histology.

### Example 2 - HPV detection as a follow-up of low grade lesions in the Swedish gynaecological screening program

In Sweden approximately 40 000 cytology cases pr. year show aberrations which needs follow-up. Most cases regress spontaneously but some progress if not treated. There is also a problem of low sensitivity for cytology in the follow-up procedure. In detection of pre-cancerous lesions, both specificity and sensitivity has been found to improve drastically when HPV testing is performed after detection of cytological ASCUS or CIN I.

The main objective was to evaluate the respective roles of HPV RNA and DNA tests in relation to cytology and histology in the Swedish screening program. Another important objective was to estimate the risk of missing CIN II+ in women with CIN I or ASCUS but negative with either HPV RNA or DNA tests.

The tested material stems from 15000 women following the normal screening program in the central part of Sweden. All women positive for ASCUS or CIN I with cytology were selected for further studies. All the cytological or histological material was re-evaluated blindly by an experienced pathologist. The samples positive for ASCUS and CIN I (N=240) were evaluated with PreTect HPV-Proofer (N=240), and a randomised selection of samples was tested by Hybrid Capture II (HCII) and cytology (N=127) and cytology alone (N=112) after 4 months. They were compared with histology from LEEP biopsies (N=126) after 7 months and with PreTect HPV-Proofer (N=240), HCII and cytology after 12 months (Table 2). All samples with ASCUS and CIN I were tested for mRNA. Coloposcopy directed LEEP biopsies (N=126) were taken as a part of the follow-up for all women with an abnormal cytology diagnosis and/or positive HPV DNA test (after 4 months). HPV DNA was detected using the HCII assay (Digene, Gatesburg, MD, USA). Identification and individual typing of E6/E7 mRNA transcripts from HPV 16, 18, 31, 33, and 45 was carried out using the PreTect HPV-Proofer assay (NorChip AS, Klokkarstua, Norway).

### Results

The results of HPV tests have been compared with cytology 4 and 12 months after and with histology diagnosis 7 months after positive cytology diagnosis. Frequency and distribution of HPV types is presented in table 3. Concordance between cytology and histology was found in 19% of cases. Cytology and the DNA test were considerably more often positive in benign and low-grade lesions by histology than the RNA test. With histology as the "golden standard", the RNA test revealed a higher positive predictive value, and higher specificity (46% and 85.3% respectively) than the DNA test (31% and 51% respectively). However, the DNA test revealed a higher sensitivity (91%) than the RNA based test (81%). 19% of the cases treated with LEEP conization showed aberrant cytology 5 months after treatment, 0.5% were found to be CIN II+. HPV DNA was detected in 24% and HPV RNA was detected in 6% of these cases (Table 2).

**Table 2: Overall results**

| | 0 month | 4 month | 7 month | 12 month |
|---|---|---|---|---|
| Cytology | 240/240 (100%) | 93/217 (43%) | Not analysed | 30/160 (19%) |
| HCII | Not analysed | 64/113 (57%) | Not analysed | 41/169 (24%) |
| Pretect | Not | 56/240 | Not | 14/231 |
| HPV-Proofer | analysed | (23%) | analysed | (6%) |
| Histology | Not analysed | Not analysed | 100/118 (85%) | Not analysed |

**Table 3: Histological results versus HPV DNA, RNA and cytological results**

| Cytological diagnosis | ASCUS | CIN I | CIN II | CIN III-ASCUS-H |
|---|---|---|---|---|
| HPV 16 | 6 | 4 | 4 | 2 |
| HPV 18 | 2 | 1 | 0 | 3 |
| HPV 31 | 0 | 2 | 1 | 1 |
| HPV 33 | 3 | 2 | 1 | 2 |
| HPV 45 | 3 | 0 | 0 | 1 |
| HPV-Proofer total | 14/49 (29%) | 9/27 (33%) | 5/8 (63%) | 6/6 (100%) |
| HCII | 20/26 (77%) | 11/15 (73%) | 3/3 (100%) | 2/2 (100%) |
| Histology CIN II+ only cyt | 3/23 (13%) | 3/12 (25%) | 4/5 (80%) | 3/4 (75%) |
| Histology CIN II+ cyt & HCII | 7/20 (35%) | 1/11 (9%) | 3/3 (100%) | 2/2 (100%) |
| Histology CIN II+ all samples | 12/45 (27%) | 4/27 (14%) | 7/8 (88%) | 5/6 (83%) |

### Discussion and conclusion

The higher positive predictive value and higher specificity of the RNA based method compared with the DNA based method may be explained by the fact that expression of the E6/E7 oncogenes is required for development and maintenance of the malignant phenotype. The risk of missing CIN II+ in women with CIN I or ASCUS, but negative with either HPV RNA or DNA tests was extremely low (0.2%), confirming the added value of HPV testing in cytological ASCUS or CIN I.

### Example 3 - High-risk HPV infections without oncogene expression in women younger than 30 years of age

Human papillomavirus (HPV) is a common virus infection among women, particularly in younger age groups, although most infections are transient and asymptomatic. In the Scandinavian countries, the HPV prevalence in the women population above 30 years of age varies between 5 and 15% and the HPV prevalence in younger women may be as high as 30-40%. Also, 70 - 80% of the sexually active women will, at some point in their lifetime, acquire an HPV infection. However, the majority of the infections will spontaneously clear out, and only a small proportion will persist and give rise to cervical intraepithelial neoplasia (CIN).

The aim of this study was to compare the detection of E6/E7 transcripts and the detection of HPV DNA in women younger than 30 years of age.

### Material and Methods:

A total of 282 cervical samples from women younger than 30 years of age (mean age 26.9) were tested. RNA and DNA were extracted using the NucliSens Extractor and E6/E7 mRNA expression from the carcinogenic HPV types 16, 18, 31, 33, and 45 was detected by the PreTect HPV-Proofer assay (NorChip AS, Klokkarstua, Norway). The presence of HPV DNA was investigated by Gp5+/6+ consensus PCR, and HPV DNA positive samples were then subjected to type specific PCR for the same 5 HPV types.

### Results:

A total of 32.6% (n=92) samples were positive for HPV DNA by Gp5+/6+ PCR, and 24.8% (n=70) were found to be of types 16, 18, 31, 33, and 45. E6/E7 mRNA from the same five HPV types was observed in only 15.2% (n=43) of the cases.

The five carcinogenic HPV types 16, 18, 31, 33, and 45 accounted for 76% (70/92) of the HPV DNA positive samples, while an E6/E7 mRNA expression was detected in 61% (43/70) of these cases.

A cytological positive result was obtained in 8/282 cases (2.8%), of which ASCUS was observed in 5/8 cases and HPV condyloma in 3/8 cases. For the ASCUS cases, HPV DNA was detected by Gp5+/6+ consensus PCR and type specific PCR in 4/5 cases, whereas only one sample was found to contain HPV mRNA. For the HPV condyloma cases, however, HPV DNA was detected by Gp5+/6+ consensus PCR in all the samples (n=3), and by type specifc PCR in 2/3 cases, while HPV E6/E7 mRNA expression was detected by PreTect HPVProofer in only 1 case.

### Discussion:

The presence of HPV in women younger than 30 years of age is higher than for older women. This is also the case for the prevalence of the five carcinogenic HPV types 16, 18, 31, 33, and 45 compared to other types.

Lack of E6/E7 transcripts may reflect an episomal state of the virus and hence a controlled regulation of the transcription process. These infections may be more likely to clear out. Integration of the virus, however, may disrupt the E2 gene, and thereby also its function as regulator of E6/E7 transcription.

### Conclusion:

In this young outpatient population, HPV infection with an oncogene expression, is detected in less than 50% of the consensus PCR positive samples. Thus, monitoring E6/E7 gene expression for HPV types 16, 18, 31, 33 and 45 may be a valuable diagnostic test in addition to cytology. mRNA detection may be more discriminatory for progressive disease in young women.

### Example 4 - DNA versus RNA based methods for HPV testing

The aims of this study were to validate two commercially available assays for HPV testing in order to investigate the prevalence of high risk HPV infections in women with negative and positive cytology and to evaluate the outcome of DNA-based and RNA-based testing compared to cytology and histology.

### Material and Methods

The study population was selected from outpatient departments and gynaecologists in private practice. Included in this study were 628 women with median age 40 years (range, 19-85). A conventional Pap smear was taken first, and the remaining material was transferred to a PreservCyt^{™} vial (Cytyc Corporation). Testing for high-risk HPV DNA (type 16, 18, 31, 33, 35, 39, 45, 51, 52, 56, 58, 59, and 68) was performed with the Hybrid Capture II assay (Digene Corporation) and individual identification of E6/E7 mRNA transcripts from HPV 16, 18, 31; 33, and 45 with the Pre Tect HPV-Proofer assay (NorChip AS), a real-time NASBA technique.

Biopsies were taken when HPV test was positive or cytology revealed HSIL. Histology was regarded as the "gold standard".

### Results

Concordance between cytology and histology were found in 53% of cases. High-grade histology (CIN 2+) was detected in 61% of the women with benign or lowgrade cytology. Kappa value was 0.31. Different outcomes of the two tests were present in 17% (109/628) of cases (table 4).

**Table 4: HPV testing related to histology in cases with different outcome of the two HPV tests**

| | | | |
|---|---|---|---|
| | Histology | | |

| HPV test | < CIN 2 | ≥ CIN 2 | Total |
|---|---|---|---|
| DNA+/RNA- | 40 | 59 | 99 |
| RNA+/DNA- | 1 | 9 | 10 |
| Total | 41 | 68 | 109 |

Both HPV tests showed significant association with grade of the lesions (p<0.001). The DNA test was more often positive in benign and low-grade lesions. The DNA test revealed higher sensitivity but lower specificity compared to the RNA test (Table 5).

**Table 5: The performance of HPV testing for detection of histological confirmed CIN 2+**

| | Sensitivity (%) | | Specificity (%) | |
|---|---|---|---|---|
| | DNA | RNA | DNA | RNA |
| Age | | | | |
| < 30 years (n=102) | 98 | 82 | 20 | 70 |
| ≥ 30 years (n=281) | 93 | 76 | 40 | 81 |
| Cytology | | | | |
| Normal (n=105) | 89 | 62 | 79 | 87 |
| Low grade (n=73) | 96 | 72 | 22 | 72 |
| High grade (n=182) | 96 | 83 | 67 | 50 |

### Conclusion

The RNA test revealed a higher prognostic value and higher specificity than the DNA test.

### Example 5 - HPV type specific DNA and RNA persistence

The course and persistence of HPV infection was analysed in 54 women who were HPV positive and free of any cytological disease using HPV genotyping with a linear array assay.

The impact of HPV infection on development of cervical cytological abnormality (dyskaryosis) was monitored by repeat HPV genotyping and cytological assessment 2 years later. Detection of HPV transcripts of known HPV oncogenes E6 and E7 using the HPV-Proofer assay was also performed at both time points.

### Materials and Methods:

Liquid based cytology (LBC) samples were obtained in 2,000 from more than 3,000 women as part of an ongoing study designed to assess HPV persistence (baseline). LBC involves rinsing a cervical specimen into a vial containing a cellular preservative solution, rather than depositing it directly on a slide as is performed during the conventional Papanicalou smear. Primary care personnel carried out specimen collection, flat layer slides were created by the ThinPrep^{®} procedure and cytological grading was performed according to British Society for Clinical Cytology guidelines. A cohort of 54 women were selected on the basis of having a cytologically normal result at baseline but who were also HPV DNA positive for at least one of the following "high-risk" HPV types: 16, 18, 31, 33 and 45, considered the most commonly found high-risk types in Europe and implicated in >90% of cancers. Women were recalled for a follow up LBC smear 2 years after baseline when cytological assessment and both HPV DNA and RNA testing was performed.

After cytology, residual cells in the LBC sample were centrifuged at 3,500 rpm for 10 min and stored as split cellular pellets at - 70°C prior to nucleic acid extraction and HPV detection. Automated DNA extraction was performed using a BioRobot 9604^{®} (QIAGEN Ltd., Crawley, UK) using the reagents supplied with the QIAamp^{®} 96 DNA Swab BioRobot^{™} Kit whereas RNA extraction was performed by application of RNeasy columns (QIAGEN Ltd.) following the protocol for isolation from animal cells, according to the manufacturer's instructions. Nucleic acid was stored at -70°C prior to HPV detection.

HPV DNA genotyping was undertaken by linear array hybridisation assay (LA) which involved the hybridisation of a 450 nt PCR amplicon generated by the PGMY primer set to nylon strip containing immobilised probes [Gravitt et al., 2000; Coutlee et al., 2002]. The strip contained two levels of β-globin control probes, 18 high-risk HPV (HR-HPV) probes; 16, 18, 26, 31, 33, 35, 39, 45, 51, 52, 55, 56, 58, 59, 68, 73, 82, 83 and 9 low-risk HPV (LR-HPV) probes: 6, 11, 40, 42, 53, 54, 57, 66, 84. PCR reagents, probe strips and developing reagents were supplied by Roche Molecular Systems, Inc. (Alameda). Any sample that tested β-globin negative would be excluded from analysis. RNA amplification was achieved via an isothermal NASBA amplification and type specific detection was performed using molecular beacon (MB) probes directed against full-length E6/E7 mRNA for HPV types HPV 16, 18, 31, 33, 45. All reagents required for NASBA amplification and HPV detection were supplied as part of the PreTect^{®} HPV-Proofer Kit, (NorChip, Klokkarstua, Norway). Fluorescent detection of accumulated mRNA product was performed in real time using a NucliSens EasyQ Analyzer and fluorescent profiles analysed using the PreTect analysis software (PAS, NorChip).

### Results:

A total of 11/54 (20%) women developed dyskaryosis after 2 years with 31/54 and 23/54 women exhibiting transient and persistent infections respectively, as monitored by DNA genotyping. Women who maintained type-specific persistent HPV infection were significantly more likely to develop dyskaryosis compared to those who exhibited a transient infection (P<0.001). The presence of HPV mRNA E6-E7 transcripts was less sensitive but more specific for the detection of disease at follow up. Moreover, women who were DNA positive and also positive for mRNA transcripts at baseline were significantly more likely to harbour persistent infection compared to those in whom DNA only was detected at baseline (P<0.013). This study highlights the importance of detecting persistent type specific HPV infection to identify those women more at risk of developing cervical abnormalities. Detection of E6/E7 transcripts encoding full length E6 protein has the potential to identify which high-risk HPV infections may persist, even when detection is performed at only a single time point without repeat testing. Detection of E6/E7 mRNA transcripts identified which infections were more likely to persist.

**Table 6. Proportion of Detectable Persistent HPV Infections in individuals With and Without Concurrent Evidence of Dyskaryosis on Follow-Up as Detected by DNA Genotyping and HPV RNA Transcript Detection**

| Cytological assessment on follow-up | No. of cases | No. of persistent infections (DNA) | No. of persistent infections (RNA) | No. of persistent infections (DNA or RNA) |
|---|---|---|---|---|
| Abnormal | 11 | 10 (90.1) | 6(54.5) | 10(90.1) |
| Normal | 43 | 13 (30.2) | 5(11.6) | 15(34.8) |

**Table 7. Comparison of DNA Genotyping and RNA Transcript Detection for the Detection of 11 Cases of Dyskaryosis**

| Method of detection | Sensitivity | | Specificity | |
|---|---|---|---|---|
| DNA | 10/11 | 90.9% | 19/43 | 44.2% |
| RNA | 8/11 | 72.7% | 35/43 | 81.4% |

### Example 6

The following tables summarize detection of full length E6/E7 mRNA transcripts by PreTect HPV-Proofer versus detection of HPV DNA by consensus and type-specific PCR in samples from an African out-patient population. Samples scored as histology (+) were assessed as CIN II+ on the basis of histology.

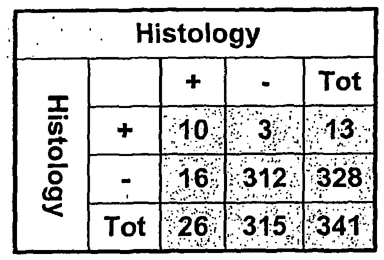

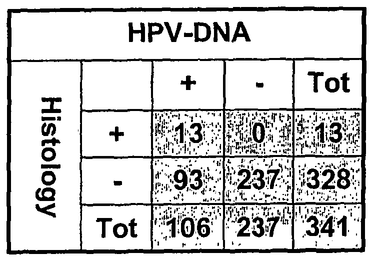

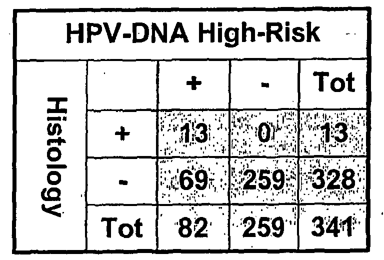

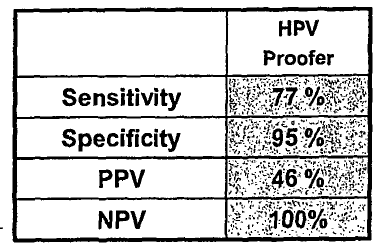

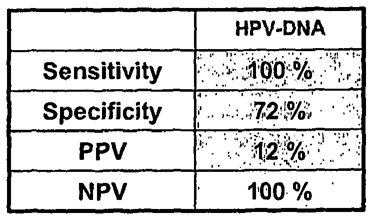

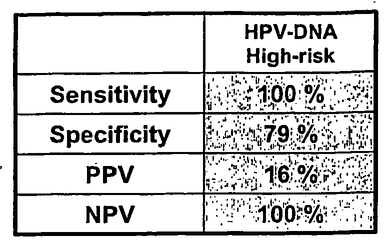

The results of this study illustrate the specificity of an assay based on detection of E6/E7 transcripts which encode full length E6 protein from any of HPV types 16, 18, 31, 33 or 45 (e.g. PreTect HPV-Proofer) for samples exhibited cell abnormalities scored as CIN II+ on the basis of histology. The RNA assay has a similar specificity to histology but higher sensitivity.

## Claims

1. An *in vitro* method of screening human subjects for the presence of persistent transforming infection with human papillomavirus in at least one cell or tissue, which method comprises screening the subject for expression of mRNA transcripts of the E6/E7 gene of human papillomavirus which encode a full length E6 protein in a test sample comprising mRNA derived from the cell or tissue, wherein subjects positive for expression of such mRNA transcripts of the E6/E7 gene of human papillomavirus are scored as having a persistent transforming infection with human papillomavirus in the cell or tissue.

2. A method according to claim 1 which comprises detecting the presence of mRNA transcripts of the E6/E7 gene of human papillomavirus using a technique which is able to detect E6/E7 mRNA from at least one cancer-associated HPV type.

3. A method according to claim 2 which comprises detecting the presence of mRNA transcripts of the E6/E7 gene of human papillomavirus using a technique which is able to detect E6/E7 mRNA from HPV types 16, 18, 31, 33.

4. A method according to claim 2 which comprises detecting the presence of mRNA transcripts of the E6/E7 gene of human papillomavirus using a technique which is able to detect E6/E7 mRNA from HPV types 16, 18, 31, 33 and 45.

5. A method according to claim 2 which comprises detecting expression of mRNA transcripts of the E6/E7 gene from any one or any combination of two or more of HPV types 16, 18, 31, 33 or 45, wherein the presence of mRNA transcripts of the E6/E7 gene of human papillomavirus from any one of the tested HPV types in the sample is taken as a positive result.

6. A method according to any one of claims 1 to 5 wherein detection of expression of mRNA transcripts of the E6/E7 gene is carried out using an amplification reaction to amplify of a region of the mRNA, together with real-time detection of the products of the amplification reaction.

7. A method according to claim 6 wherein detection of expression of mRNA transcripts of the E6/E7 gene is carried out using real-time NASBA.

8. A method according to claim 7 wherein detection of expression of mRNA transcripts of the E6/E7 gene is carried out using the Pre-Tect HPV-Proofer™ assay kit.

9. A method according to any one of claims 1 to 8 wherein the human subjects are subjects previously identified as infected with human papillomavirus DNA, preferably in the cell or tissue under test.

10. A method according to any one of claims 1 to 9 wherein the human subjects are subjects having a previous diagnosis of ASCUS, CIN 1 lesions or condyloma.

11. A method according to any one of claims 1 to 9 when used for primary screening of individuals who have no previous diagnosis of cervical abnormalities by cytology.

## Patentansprüche

1. In vitro Verfahren zum Screening von menschlichen Individuen für die Anwesenheit einer persistierenden transformierenden Infektion mit humanem Papillomvirus in mindestens einer Zelle oder einem Gewebe, wobei das Verfahren Screening des Individuums für die Expression von mRNA-Transkripten des E6/E7-Gens von humanem Papillomvirus, die ein Volllängenprotein E6 kodieren, umfasst, in einer Testprobe, umfassend mRNA, die aus der Zelle oder dem Gewebe stammt, wobei die Individuen positiv für die Expression solcher mRNA-Transkripte des E6/E7-Gens von humanem Papillomvirus so bewertet werden, eine persistierende transformierende Infektion mit humanem Papillomvirus in der Zelle oder dem Gewebe aufzuweisen.

2. Verfahren nach Anspruch 1, das Detektieren der Anwesenheit von mRNA-Transkripten des E6/E7-Gens von humanem Papillomvirus unter Verwendung einer Technik umfasst, die fähig ist, E6/E7-mRNA aus mindestens einem Krebs-assoziierten HPV-Typ zu detektieren.

3. Verfahren nach Anspruch 2, das Detektieren der Anwesenheit von mRNA-Transkripten des E6/E7-Gens von humanem Papillomvirus unter Verwendung einer Technik umfasst, die fähig ist, E6/E7-mRNA aus den HPV-Typen 16, 18, 31, 33 zu detektieren.

4. Verfahren nach Anspruch 2, das Detektieren der Anwesenheit von mRNA-Transkripten des E6/E7-Gens von humanem Papillomvirus unter Verwendung einer Technik umfasst, die fähig ist, E6/E7-mRNA aus den HPV-Typen 16, 18, 31, 33 und 45 zu detektieren.

5. Verfahren nach Anspruch 2, das Detektieren der Expression von mRNA-Transkripten des E6/E7-Gens aus irgendeinem oder irgendeiner Kombination von zwei oder mehreren HPV-Typen 16, 18, 31, 33 oder 45 umfasst, wobei die Anwesenheit von mRNA-Transkripten des E6/E7-Gens von humanem Papillomvirus aus irgendeinem der getesteten HPV-Typen in der Probe als ein positives Ergebnis genommen wird.

6. Verfahren nach einem der Ansprüche 1 bis 5, wobei die Detektion der Expression von mRNA-Transkripten des E6/E7-Gens unter Verwendung einer Amplifikationsreaktion durchgeführt wird, um eine Region der mRNA zu amplifzieren, zusammen mit Echtzeit-Detektion der Produkte der Amplifikationsreaktion.

7. Verfahren nach Anspruch 6, wobei die Detektion der Expression von mRNA-Transkripten des E6/E7-Gens unter Verwendung von Echtzeit-NASBA durchgeführt wird.

8. Verfahren nach Anspruch 7, wobei die Detektion der Expression von mRNA-Transkripten des E6/E7-Gens unter Verwendung des Pre-Tect HPV-Proofer^{™}-Assaykits durchgeführt wird.

9. Verfahren nach einem der Ansprüche 1 bis 8, wobei die menschlichen Individuen Individuen sind, die vorher identifiziert wurden, mit humaner Papillomvirus-DNA infiziert zu sein, bevorzugt in der Zelle oder dem Gewebe, die/das getestet wird.

10. Verfahren nach einem der Ansprüche 1 bis 9, wobei die menschlichen Individuen Individuen sind, die eine vorherige Diagnose von ASCUS, CIN-1-Läsionen oder Condylom aufweisen.

11. Verfahren nach einem der Ansprüche 1 bis 9, wenn es für primäres Screening von Individuen verwendet wird, die keine vorherige Diagnose von Gebärmutterhals-Abnormalitäten durch Zytologie aufweisen.

## Revendications

1. Procédé *in vitro* pour tester des sujets humains en ce qui concerne la présence d'une infection transformante persistante par le papillomavirus humain dans au moins une cellule ou au moins un tissu, procédé qui comprend l'étape consistant à tester le sujet en ce qui concerne l'expression de transcripts d'ARNm du gène E6/E7 du papillomavirus humain codant pour une protéine E6 complète dans un échantillon d'essai comprenant de l'ARNm dérivé de la cellule ou du tissu, dans lequel les sujets positifs pour l'expression de ces transcripts d'ARNm du gène E6/E7 du papillomavirus humain sont classés en tant que sujets présentant une infection transformante persistante par le papillomavirus humain dans la cellule ou le tissu.

2. Procédé suivant la revendication 1, qui comprend la détection de la présence de transcripts d'ARNm du gène E6/E7 du papillomavirus humain en utilisant une technique qui permet de détecter l'ARNm E6/E7 à partir d'au moins un type de HPV associé au cancer.

3. Procédé suivant la revendication 2, qui comprend la détection de la présence de transcripts d'ARNm du gène E6/E7 du papillomavirus humain en utilisant une technique qui permet de détecter l'ARNm E6/E7 des types de HPV 16, 18, 31, 33.

4. Procédé suivant la revendication 2, qui comprend la détection de la présence de transcripts d'ARNm du gène E6/E7 du papillomavirus humain en utilisant une technique qui permet de détecter l'ARNm E6/E7 des types de HPV 16, 18, 31, 33 et 45.

5. Procédé suivant la revendication 2, qui comprend la détection de l'expression de transcripts d'ARNm du gène E6/E7 de n'importe quel type ou de n'importe quelle combinaison de deux ou plus de deux types de HPV 16, 18, 31, 33 ou 45, dans lequel la présence de transcripts d'ARNm du gène E6/E7 du papillomavirus humain provenant de n'importe lequel des types de HPV testés dans l'échantillon est considérée comme étant un résultat positif.

6. Procédé suivant l'une quelconque des revendications 1 à 5, dans lequel la détection de l'expression de transcripts d'ARNm du gène E6/E7 est effectuée en utilisant une réaction d'amplification pour amplifier une région de l'ARNm, conjointement avec la détection en temps réel des produits de la réaction d'amplification.

7. Procédé suivant la revendication 6, dans lequel la détection de l'expression de transcripts d'ARNm du gène E6/E7 est effectuée en utilisant la technique NASBA en temps réel.

8. Procédé suivant la revendication 7, dans lequel la détection de l'expression de transcripts d'ARNm du gène E6/E7 est effectuée en utilisant le kit analytique Pre-Tect HPV-Proofer^{™}.

9. Procédé suivant l'une quelconque des revendications 1 à 8, dans lequel les sujets humains sont des sujets identifiés auparavant en tant que sujets infectés par de l'ADN de papillomavirus humain, de préférence dans la cellule ou le tissu à l'essai.

10. Procédé suivant l'une quelconque des revendications 1 à 9, dans lequel les sujets humains sont des sujets ayant un diagnostic antérieur de ASCUS, de lésions CIN 1 ou de condylome.

11. Procédé suivant l'une quelconque des revendications 1 à 9, lorsqu'il est utilisé pour le test primaire d'individus n'ayant aucun diagnostic antérieur d'anomalies du col de l'utérus par cytologie.
